(19) Europäisches Patentamt European Patent Office Office européen des brevets

(11) **EP 4 479 795 B1**

(12) **EUROPEAN PATENT SPECIFICATION**

(45) Date of publication and mention
of the grant of the patent:
**02.04.2025 Bulletin 2025/14**

(21) Application number: **23703809.6**

(22) Date of filing: **13.02.2023**

(51) International Patent Classification (IPC):
*G02C 7/02* (2006.01)    *A61B 3/00* (2006.01)
*G02C 13/00* (2006.01)    *A61B 3/113* (2006.01)

(52) Cooperative Patent Classification (CPC):
**G02C 13/005; A61B 3/0091; A61B 3/113;**
**G02C 7/027;** A61B 3/005

(86) International application number:
**PCT/EP2023/053504**

(87) International publication number:
**WO 2023/152372 (17.08.2023 Gazette 2023/33)**

(54) **METHOD AND MOBILE DEVICE FOR DETERMINING A VISUAL POINT OF A PERSON**

VERFAHREN UND MOBILE VORRICHTUNG ZUR BESTIMMUNG EINES SICHTPUNKTES EINER
PERSON

PROCÉDÉ ET DISPOSITIF MOBILE POUR LA DÉTERMINATION D'UN POINT VISUEL D'UNE
PERSONNE

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **14.02.2022 EP 22156628**

(43) Date of publication of application:
**25.12.2024 Bulletin 2024/52**

(73) Proprietors:
• **Carl Zeiss Vision International GmbH**
**73430 Aalen (DE)**
• **Carl Zeiss Vision Ireland Ltd.**
**Wexford (IE)**

(72) Inventors:
• **WAHL, Siegfried**
**73072 Donzdorf (DE)**
• **KRATZER, Timo**
**73434 Aalen (DE)**
• **KALTENBACHER, Axel**
**73432 Aalen (DE)**
• **LEUBE, Alexander**
**73431 Aalen (DE)**
• **WEINREICH, Manuela**
**73431 Aalen (DE)**
• **SINNOTT, David**
**Wexford, Y35K7T8 (IE)**
• **BREHER, Katharina**
**72070 Tübingen (DE)**

(74) Representative: **Kraus & Lederer PartGmbB**
**Thomas-Wimmer-Ring 15**
**80539 München (DE)**

(56) References cited:
**EP-B1- 3 413 122        US-A1- 2020 349 754**
**US-A1- 2021 142 566    US-A1- 2021 181 538**
**US-B2- 10 330 958       US-B2- 9 645 413**
**US-B2- 9 841 615**

**Description**

The present application relates to methods and mobile devices for determining a visual point of a person, as well as to corresponding computer programs and methods for manufacturing spectacle lenses based on the determined visual point.

For adapting spectacle lenses to a particular spectacle frame for a particular person, various parameters are conventionally determined, which are referred to as centration parameters. Centration parameters are parameters that are needed to be correctly arranged, that is to say centered, of spectacle lenses in a spectacle frame such that the spectacle lenses are worn in a correct position relative to the person's eyes.

Examples of such centration parameters comprise the interpupillary distance, the vertex distance, the face form angle, the y coordinates of the left and right centration points (also designated as the fitting point height), the distance visual point, the pantoscopic angle, and further parameters defined in section 5 of DIN EN ISO 13666:2012 and, among other things, the inclination of the frame.

For further designing and manufacturing spectacle lenses adapted to a particular person, the individual eye behavior for seeing in different distances is important. This eye behavior depends on the viewing distance itself, the scene, and convergence of the eye. The eye behavior determines where the person wearing the spectacle looks through the spectacle lens. According to DIN EN ISO 13666:2012 5.11, the intersection of the line of sight of the person when looking at a particular target with the back surface of the spectacle lens is referred to as visual point which corresponds to the aperture in the spectacle plane. In the context of the present application, sometimes the thickness of the spectacle lens and/or a curvature of the spectacle lens may be neglected, such that the visual point is taken as the intersection of the line of sight with a plane approximating the position of the spectacle lens.

Knowing the visual point for different distances, for example the distance visual point according to 5.16 of DIN EN ISO 13666:2012 or the near visual point according to 5.17 of DIN EN ISO 13666:2012 is particularly important for design and centration of progressive addition lenses (PALs), where different parts of the lens provide optical corrections for different distances, for example for near vision or far vision. Distance visual point is an assumed position of the visual point on a lens, which is used for distance vision under given conditions and near vision point is the same used for near vision. The near vision part of the lens should be for example at or around the near visual point, whereas a distance vision part of the lens should be located at or around the distance visual point. Also, for single vision lens (SVL) design and centration knowing the visual point(s) may be helpful.

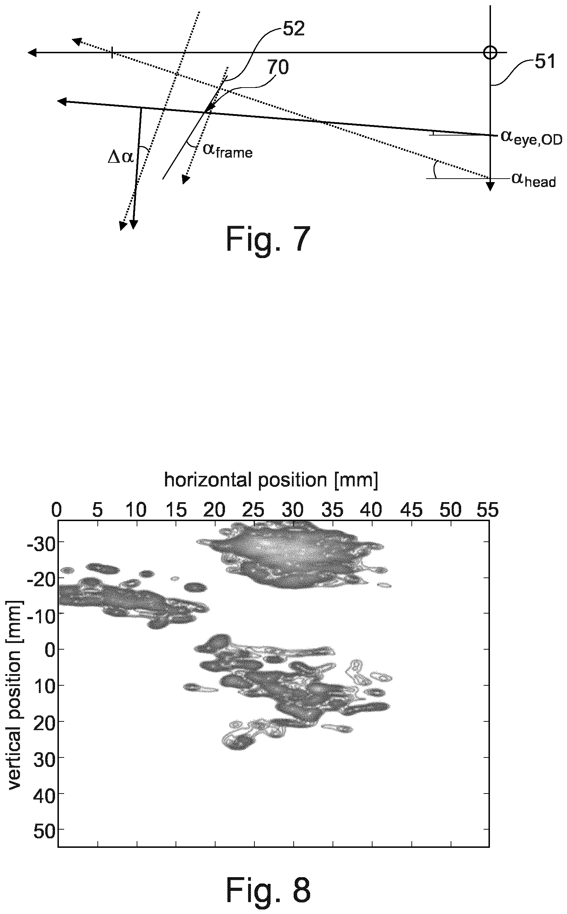

To illustrate, FIG. 8 shows an example distribution of visual points over a field of view of a lens for a single vision lens in form of a heat map representing where the visual points are during a typical office task including writing, reading and switching between two screens, monitored over a certain time. In other words, the visual points, i.e. intersection between the line of sight of the person and the spectacle lens, were determined and plotted in the diagram of Fig. 8 over time. Here, when the person looks at different targets, the person may either move the eye, leading to different visual points, or move the head, such that the visual point stays constant while the head moves, or combinations thereof (head movement in combination with a slight eye movement, for example). In the example shown, there is a preferred visual point area between about 20 and 40 mm horizontal and -20 and -35 mm vertical, with visual points being detected in some other areas over the field of view. This means that the particular person wearing this particular spectacle mostly looks through the preferred visual point area, but may move the eye to look through other parts of the lens sometimes.

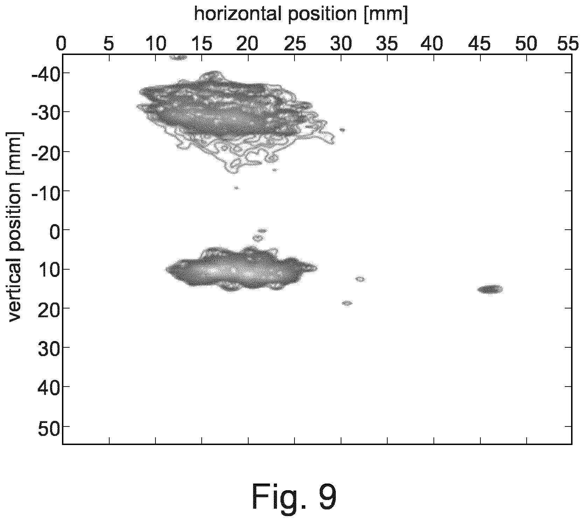

FIG. 9 shows an example distribution of the visual points for a progressive addition lens for the same task as in FIG. 8. Here, two main visual point areas may be seen, one for far sight and the other for near sight. The areas for far sight and near sight in progressive addition lenses are generally limited compared to the whole area of single vision lens, such that apart from changing between far field and near field which is usually done by eye movement, more head movement is required. Therefore, it has been found in studies that generally persons wearing single vision lenses use more eye movement, i.e., are more "eye movers" than person wearing progressive addition lenses, who are more "head movers".

The location and distribution of the visual points depend not only on the type of the lens (PAL or SVL) but also on the person wearing the lens. For example, a person may be more of an eye mover or more of a head mover. Therefore, knowing the visual point or visual point distribution for specific tasks (like reading and writing) of a person is helpful for producing a lens-like progressive addition lens specifically adapted to the person wearing the spectacle.

Various systems are known for measuring individual vision parameters including the vision point or vision point distribution of a person and designing lenses accordingly, for example Essilor Visioffice 2 (https://www. pointsdevue.com/article/effect-multifocal-lense s-eye-and-head-movementspresbyopic-vdu-users-neck-and-shoulder), Essilor Visioffice X (https://www. pointsdevue.com/white-paper/varilux-x-seriestm-len ses-near-vision-behaviorpersonalization) or Essilor Visiostaff (https://www.essilorpro.de/Produkte/Messe n-und-Demonstrieren/visioffice/Documents/Visiostaff_ Broschuere.pdf).

Some techniques used in Visioffice 2 are described in EP 3145386 B1. This document generally describes a method for determining at least one parameter of visual behavior of a person. The center of rotation of at least one eye is determined in a first reference frame associated with the head of the person. Then, in a real-life situation like reading, an image of the person which includes a body part of the person is captured, and the position and orientation of the body part is determined in a second reference frame. Ultimately, the center of rotation of the eye is then determined in the second reference frame. Based on knowing the center of rotation, eye parameters are determined. By mounting an eye tracker to a pair of spectacles used by the individual, a visual point may be determined. This application requires an eye tracker on the spectacle frame.

A somewhat similar system is disclosed in WO 2015/124574 A1, which also uses an eye tracker mounted on a spectacle frame. With this spectacle frame mounted eye tracker, eye movement of a person may be observed over a longer time. However, this requires the person to wear the corresponding spectacle frame with the eye tracker.

Therefore, the above solutions require an eye tracker mounted to the spectacle frame, i.e., specific hardware.

EP 1815289 B9 discloses a method for designing a spectacle lens, considering a movement of head and eye of a person. For determining the visual point, a commercially available head and eye movement measurement device is modified, such that also here a specific hardware is needed.

EP 1747750 A1 discloses a further method and device for determining the visual behavior of a person, for example the visual point, for customization of a spectacle lens. The person has to wear a specific hairband with LEDs for tracking the head movement, which may be cumbersome to the person.

EP 2342599 B1 and EP 1960826 B1 each disclose a determination of the visual behavior of a person based on manual measurements, in the former via convergence data and the latter via wire had a head inclination. Therefore, the various solutions discussed above require specific equipment. Some cases which use eye and head tracking devices can be used reliably only in laboratory set ups, or require manual measurements which are time consuming.

US 2020 / 349 754 A1 discloses a method of generating a 3D model of a head using a smartphone, where a depth map is used.

US 2021 / 142 566 A1 discloses a virtual try-on of spectacle frames.

US 9 645 413 B2 and US 9 841 615 B2 discuss approaches for determine vision points.

EP 3413122 A1 discloses a method and a device for determining a near field visual point, where in some implementations a device like a tablet PC may be used for measuring the near vision point. The device may be used in connection with a centration device. The position and/or orientation of a near target like the above-mentioned tablet is determined, and an image of a person as captured while the person looks at the target is taken. The near visual point is then determined based on the image, position and/or orientation. This method essentially uses a static measurement for a single position. In some implementations, an avatar, i.e., a model, of the head may be used to which a virtual spectacle frame is fitted, and the vision point is determined using this avatar fitted with the virtual spectacle frame. For generating the avatar, a stationary device with a plurality of cameras provided approximately in a semicircle is used. Therefore, while a mobile device like a tablet is involved, for making use of an avatar still a specific stationary apparatus needs to be provided. Furthermore, only a single near vision point is determined. Additionally, a far vision point may be determined.

US 2021 / 181 538 A1 discloses a further method where an avatar is used to determine a visual point, with only a single visual point being detected.

Starting for example from EP 3413122 A1, it is an object of the present invention to provide a flexible approach to determine the visual point, which may use virtual spectacle frames, but does not require specific hardware, and enables measurement of a vision point distribution. Compared to the other references cited above, no specific hardware and no manual measurements which requires a trained person like an optician would not be necessary.

According to a first aspect of the invention, a computer-implemented method for a mobile device including at least one sensor, the at least one sensor comprising an image sensor, to determine a visual point of a person, wherein the visual point is an intersection of a line of sight of the person with a plane approximating the position of a spectacle lens, is provided, comprising:

determining a head position and a pupil position of the person while the person looks at a target using the at least one sensor, and determining the visual point based on an avatar, which is a 3D model of at least an eye portion of the head of the person, which is set to the determined head and pupil position, based on the set head and pupil positions of the avatar and on the position of the plane approximating the position of the spectacle lens, wherein either:

- the method further comprises virtually fitting a spectacle frame to the avatar, and determining the visual point based on the virtually fitted spectacle frame; or
- the person wears a spectacle frame, and the method further comprises: identifying the spectacle frame worn by the person, wherein determining the visual point is further based on the identified spectacle frame in that

determining the head position comprises determining parts or facial landmarks of the head of the person, and characterized in that the method further comprises moving the target, wherein determining the visual point is performed for a plurality of target positions, and in that the target is the mobile device. The method may be implemented using an off the shelf mobile device like a smartphone or a tablet PC equipped with corresponding sensors. In this case, a computer program for such a mobile device may be provided to implement any of the methods above. In the context of mobile devices, such a computer program is often referred to as application or "app".

According to another aspect, the mobile device provided, comprising:

at least one sensor, comprising an image sensor and configured to determine a head position and a pupil position of a person while the person looks at a target, and a processor configured to determine the visual point of the person based on an avatar which is a 3D model of at least an eye portion of the person and set to the determined head and pupil position based on the set head and pupil positions of the avatar and on a position of a plane approximating the position of a spectacle lens, wherein the visual point is an intersection of a line of sight of the person with a plane approximating the position of the spectacle lens, wherein either:

- the processor is further configured to virtually fit a spectacle frame to the avatar, and determine the visual point based on the virtually fitted spectacle frame; or
- the person wears a spectacle frame, and the processor is further configured to identify the spectacle frame worn by the person, wherein determining the visual point is further based on the identified spectacle frame, in that

determining the head position comprises determining parts or facial landmarks of the head of the person, and characterized in that the mobile device is further configured to determine the visual point of the person for a plurality of positions while the target moving through a field of view of the person, and in that the target is the mobile device.

The mobile device may be configured, for example programmed, for any of the methods discussed.

A processor, in this respect, refers to any entity that is capable of performing corresponding calculations, for example a microprocessor programmed accordingly, or a microcontroller. As mentioned previously, the mobile device may be an off the shelf mobile device like a smartphone or a tablet, where corresponding sensors or processors are usually provided. The sensor includes an image sensor and may for example include a depth sensor like a time-of-flight sensor.

The method is characterized in that the target, which is the mobile device, is moved through a certain field of view of the person, and the visual point is determined for a plurality of positions of the target. In this way, the visual point for a plurality of target positions may be determined. From the determined visual point of a plurality of target positions, it may also be detected if the person is a head-mover or an eye-mover as explained initially, i.e., if the person rather moves the head for following the target when it moves or only moves the eyes. The moving of the target may be performed by the person or another person, for example upon instructions output by the mobile device.

A mobile device, as used herein, refers to a device that is designed to be carried around easily. Typically, such a mobile device has a weight of less than 2kg, usually less than 1kg or even less. Examples for such mobile devices include smartphones or tablet PCs. Nowadays, smartphones or tablet PCs besides a processor and memory, include a plurality of additional components, in particularly sensors, which are used to implement the method. For example, nowadays smartphones or tablet PCs essentially always include cameras and, in many cases, also include depth sensors like time-of-flight sensors, that may be used for determining the head position and pupil position and to generate the avatar. Further, such depth sensors enable smartphone software to detect facial features and/or landmarks in an image and output a 3D-coordinate of these landmarks. For example, the head position may be determined using a depth sensor measuring the distance of different parts and/or facial landmarks of the head from the mobile device, and the pupil position may be determined using a camera of the mobile device, for example as described in the above cited EP 3413122 A1, or may also be detected as facial landmarks. Detection of facial landmarks may for example be performed as described in Wu, Y., Hassner, T., Kim, K., Medioni, G., & Natarajan, P. (2017), Facial landmark detection with tweaked convolutional neural networks, IEEE transactions on pattern analysis and machine intelligence, 40(12), 3067-3074; Perakis, P., Passalis, G., Theoharis, T., & Kakadiaris, I. A. (2012), "3D facial landmark detection under large yaw and expression variations," IEEE transactions on pattern analysis and machine intelligence, 35(7), 1552-1564; or Wu, Y., & Ji, Q. (2019), Facial landmark detection: A literature survey. International Journal of Computer Vision, 127(2), 115-142 using computer vision or machine learning techniques.

Alternatively, only an image sensor may be provided, and the landmarks may be detected in an image. The head position and pupil position may then be detected based on the images and the landmarks detected therein, for which various mobile device operating systems offer built-in solutions, for example ARKit for iOS devices and ARCore for Android devices. These solutions use trained machine learning algorithms which are able to determine 3D coordinates from 2D contours of faces in images. While the use of a depth sensor is preferred and may result in more precise measurements, for mobile devices without a depth sensor this is an alternative solution.

Therefore, the method may be implemented by programming an off-the-shelf mobile device accordingly (with a so-called "app"), and requires no specific hardware. After the avatar is generated using at least one camera and/or at least one depth sensor, the relative change in position of the head, the pupils and the facial landmarks are used to calculate a movement of the user in respect to the mobile device.

An avatar, as generally understood in the field of computing, is a graphical representation of a person. In the context of the present application, the term avatar is to be understood as a 3D avatar, all in other words a three-dimensional model representation of a person or at least an eye portion of the head of the person. Such a 3D avatar is also referred to as 3D model.

A model, in particular a 3D model, should be understood to mean a representation, in the case of a 3D model, a three-dimensional representation, of real objects which are present as a data set in a storage medium, for example a memory of a computer or a data carrier. By way of an example, such a three-dimensional representation can be a 3D mesh, consisting of a set of 3D points, which are also referred to as vertices, and connections between the points, which connections are also referred to as edges. In the simplest case, these connections form a triangle mesh. Such representation as a 3D mesh only describes the surface of an object and not the volume. The mesh need not necessarily be closed. Thus, if a head, for example, is described in the form of a mesh, it appears like a mask. Details in respect of such 3D models are found in Rau J-Y, Yeh P-C, "A SemiAutomatic Image-Based Close Range 3D Modeling Pipeline Using a Multi-Camera Configuration," Sensors (Basle, Switzerland), 2012;12(8):11271-11293. doi:10.3390/s120811271; in particular in page 11289, "FIG.16."

A voxel grid, which represents a volume-type representation, is a further option for representing a 3D model. Here, the space is divided into small cubes or cuboids, which are referred to as voxels. In the simplest case, the presence or absence of the object to be represented is stored in the form of a binary value (1 or 0) for each voxel. In the case of an edge length of the voxels of 1 mm and a volume of 300 mm x 300 mm x 300 mm, which represents a typical volume for a head, a total of 27 million voxels are consequently obtained. Such voxel grids are described in, e.g., M. Nießner, M. Zollhöfer, S. Izadi, and M. Stamminger, "Real-time 3D reconstruction at scale using voxel hashing," ACM Trans. Graph. 32, 6, Article 169 (November 2013), available at the url doi.org/1 0.1145/2508363.2508374.

An avatar of at least the eye portion of the head means that at least the eyes of the person and their surroundings are included in the avatar. In some embodiments, the avatar may for example be an avatar of the complete face of the person or of the complete head of the person, and may include more parts of the person as long as the eye portion is included.

Using the avatar, only a few landmarks of the head have to be determined during the determination of the head position, such that the avatar can be set based on the determined head position and/or facial landmarks. Similarly, as the avatar includes the eyes, determining the pupil position may be easier. Finally, by using an avatar, the method may be implemented without the person having to wear specific devices, and without the use of specific eye tracking equipment for example in a frame.

In one alternative, the person wears a spectacle frame. Here, pursuant to DIN EN ISO 7998 and DIN EN ISO 8624, a spectacle frame should be understood to mean a frame or a holder by means of which spectacle lenses can be worn on the head. In particular, the term as used herein also includes rimless spectacle frames. In this case, the method includes detecting the spectacle frame and determining the visual point based on the detected spectacle frame. Detecting a spectacle frame is for example described in EP 3 542 211 A1. In another alternative, the avatar is provided with a virtual spectacle frame in a so-called virtual try on, referred to as virtual fitting. Such a virtual try on is for example described in US 2003/0123026 A1, US 2002/105530 A1 or US 2016/0327811 A1. In this case, the method comprises determining the visual point based on the corresponding virtual spectacle frame fitted to the avatar. Using such a virtual spectacle frame has the advantage that the visual point may be determined for a plurality of spectacle frames without the person actually having to wear the spectacle frame.

Determining the head position and the pupil position of the person may be performed relative to the target. In this way, based on the avatar set to the determined head and pupil position relative to the target, a line of sight of the person may be determined, and an intersection of the line of sight with a spectacle lens plane may be taken as the visual point.

The avatar set to the determined eye and pupil position means that the avatar is set to a position where the eye and pupil positions of the avatar match the determined eye and pupil positions.

The method preferably may be further characterized by generating the avatar by the mobile device.

Therefore, unlike the approach of EP 3413122 A1, the mobile device is used for generating the avatar. This has the advantage that no specific stationary apparatus is needed. Also, compared to other approaches discussed above, no specific hardware is needed. Generating the avatar comprises providing a plurality of combined 2D and depth images of the head of the person captured from different positions, determining landmark points for each of the combined 2D and depth images, and performing a pairwise coarse registration between pairs of combined 2D and depth images based on the landmark points, after the pairwise coarse registration, performing a fine registration of the coarse registered combined 2D and depth images based on full point clouds represented by the combined 2D and depth images, and generating the avatar based on the registration. Providing the plurality of combined 2D and depth images of the head of the person comprises measuring the head of the person in a plurality of positions by the at least one sensor.

In some embodiments, for providing the avatar, the person may rotate the head about the longitudinal axis (up-down direction) of the head while the mobile device measures the head with a depth sensor and optionally also with an image sensor of a camera.

Combined depth and 2D camera images captured in this way will be simply referred to as combined images hereinafter, and in case of color camera images, it is also referred to as RGB-D images. In other embodiments, additionally or alternatively the head may be rotated about another axis like a horizontal axis. For determining the avatar, the head may be held in an upright position and the gaze may be directed horizontally far away (normal gaze direction). In this way, an individual avatar of the person may be determined without the need for additional equipment.

One main issue for generating an avatar using a smartphone is that in contrast to using a stationary apparatus like in EP 3413122 A1, the camera poses relative to the head when capturing the head with the depth sensor and camera are not known, while in a stationary arrangement the poses of the cameras are known by design of the apparatus. Generally, the term pose refers to the combination of position and orientation, as for example defined in DIN EN ISO 8373: 2012-03.

As the way a 3D object like a head is imaged onto an image sensor or captured by a depth sensor is determined by the characteristics of the device like focal length of a lens or sensor resolution, which are known by design, the problem of determining the camera poses is equivalent to the problem of registering the above-mentioned combined images.

Generally, a combined image refers to a 2D image and a depth image taken from the respective position essentially simultaneously. The 2D image may be a color image like an RGB image (red, green, blue) or may also be a grayscale image. A depth image provides a map of distances of the camera to the object, in this case, the head. For capturing the 2D part of the combined image, any conventional image sensor, combined with corresponding camera optics may be used. To capture depth images, also any conventional depth sensor like a time-of-flight sensor may be used. The combined image may include two separate files or other data entities, where in one data entity for each 2D coordinate, e.g. pixel, a greyscale value or color value is given, and in another data entity for each 2D coordinate a depth value is given. The combined image may also include only a single data entity, where for each 2D coordinate both greyscale/color information and depth information is given. In other words, the way the information is stored in data entities like files is not important as long as for the scene captured in the image both greyscale/color information and depth information is available. A camera adapted for capturing combined images, in this case color images, is also referred to an RGBD camera (red, green, blue, depth). Some modern smartphones or other mobile devices are equipped with such RGBD cameras. In other cases, also an RGBD camera or a depth sensor (which is then used together with a built-in camera of the smartphone) may be attached to a smartphone. It should be noted that the depth image need not have the same resolution as the 2D image. In such a case, a scaling operation may be performed (downscaling or upscaling) to adapt the resolution of the 2D and depth images to each other. The result is essentially a point cloud where each point has a 3D coordinate based **on the 2D** coordinates in the image and a depth coordinate from the depth sensor, as well as a pixel value (color or grayscale value).

Image registration generally relates to the process of finding a transformation which transforms one of the combined images to another one of the combined images. Such a transformation may include a rotation component, a translation component and a magnification component (magnification greater or smaller than one) and may be written in matrix form. As mentioned above, performing the registration by determining the above-mentioned transformation is essentially equivalent to determining the (relative) camera poses (positions and orientation) from which the combined images were captured.

In some embodiments, such a registration may include a pairwise coarse registration based on 3D landmark points obtained based on the combined images and a fine registration based on full point clouds represented by the combined images.

A landmark point is a predefined point on the head. Such landmark points may for example include the tip of the nose, points on the nose bridge, corners of the mouth or of the eyes, points describing the eyebrows and the like. Such landmark points in the combined 2D and depth images may be determined by various conventional means. For example, a trained machine learning logic like a neural network may be used to determine the landmark points. In this case, for training, a number of combined 2D and depth images from different positions and for a plurality of different heads are used as training data, where the landmark points may be manually annotated. After training, the trained machine learning logic determines the landmark points. Details may be found for example in Wu, Y., Hassner, T., Kim, K., Medioni, G., & Natarajan, P. (2017), Facial landmark detection with tweaked convolutional neural networks, IEEE transactions on pattern analysis and machine intelligence, 40(12), 3067-3074; Perakis, P., Passalis, G., Theoharis, T., & Kakadiaris, I. A. (2012), 3D facial landmark detection under large yaw and expression variations, IEEE transactions on pattern analysis and machine intelligence, 35(7), 1552-1564; or Wu, Y., & Ji, Q. (2019), Facial landmark detection: A literature survey, International Journal of Computer Vision, 127(2), 115-142 (2018).

The pairwise coarse registration provides a coarse alignment between the 3D landmarks of the pairs. In embodiments, this pairwise coarse registration estimates a transformation matrix between the landmarks of the two combined images that aligns the landmarks in a least square sense, i.e. that the error

$$e = \left\| L_i^{j=1} - T_1^2 L_i^{j=2} \right\|^2$$ is minimized, where $L_i^j$

is the i-th landmark of the j-th image and $T_1^2$ is a transformation matrix from a second image of the respective pair (j=2) to the first image of the respective pair (j=1). This coarse registration may be performed by a method called point a to point ICP ("Iterative Closest Point"), which is for example described in Besl, Pazl J. and McKay, Neil D., "Method for registration of 3D shapes", Sensor fusion IV: control paradigms and data structures, Vol. 1611, International Society for Optics and Photonics, 1992. Preferably, to eliminate potential outliers which may be generated in the landmark determining step, a random sampling consensus procedure may be used, as described in Fischler, Martin A., and Bolles, Robert C., "Random sampling consensus: a paradigm for model fitting with applications to image analysis and automated cartography", Communications of the ACM 24.6 (1981): 381-2395. The above and other formula presented herein use so-called homogeneous coordinates, as frequently done the case in computer vision applications. This means that transformations T are represented as 4x4 matrices [R t; 0 0 0 1], with a 3x3 rotation matrix R, a translation vector t and the last row being 0 0 0 1. 3D point (x, y, z) are augmented with a homogeneous component w, i.e. (x, y, z, w), where usually w=1. This makes it possible to include translation and rotation in a single matrix multiplication, i.e., instead of x2=R x1 + t, with x2 and x1 vectors in Cartesian coordinates, one can write x2w=T x1w, where x1w and x1w are corresponding vectors in homogeneous coordinates. Nevertheless, this is merely a matter of notation, and the same calculations may also be performed in cartesian or other coordinates.

The fine registration refines the above-mentioned transformations, i.e., makes the transformations more precise. For this fine registration, full point clouds represented by the combined 2D and depth images (i.e., the full RGBD images or point clouds derived therefrom) may be used. In particular, also color information may be used. As a coarse registration already has been performed, the fine registration may be performed more efficiently than in cases where only the point cloud is used for registration, or a corresponding mesh is used.

Different approaches may be used for fine registration. In some embodiments, the approach selected may depend on the error remaining after the coarse registration, for example the above-mentioned error e or other error quantity like angle opposition difference between the landmark points of the respective pairs of combined 2D and depth images based on the transformation determined after coarse registration. For example, also an angle difference opposition difference may be used. If this deviation, (for example error e below a value, angle difference between below a threshold angle like 5° or position difference below a position like 5 cm, for example as an average for the landmark points, RGBD odometry may be used for fine registration, where not only the depths coordinate but also the color of the points of the point cloud is considered. RGBD odometry is for example described in Park, Jaesik, Zhou, Quian-Yi and Koltun, Vladlen, "Colored point cloud registration revisited", Proceedings of the IEEE International Conference on Computer Vision 2017. For larger differences a point to plane ICP on the point clouds may be used to register the images, as described for example in Rusinkiewicz, Szymon, and Levoy, Marc "Efficient variants of the ICP algorithm", Proceedings of the third international conference on 3D digital imaging and modeling, IEEE 2001.

For both alternatives, preferably the registration is performed two times, once for estimating a transformation from a first combined image of the respective pair to a second combined image of the respective pair and once for estimating a transformation from the second combined image to the first combined image, with slightly different start values for the algorithm. This may help to increase overall accuracy. In other words, $T_1^2$ and $T_2^1$ are determined. The error between the two registrations $T_e = T_1^2 \, T_2^1$ is determined, which, if the registration is stable, should be close to an identity I4 (i.e., a diagonal matrix with only values of one). If the error, i.e., deviation from identity, is below a certain threshold, the respective transformation may be added to a so-called pose graph as an adjective between the respective combined images. In some embodiments, covariances $\Sigma_i^j$ of the transformations may also be determined.

Preferably, for both coarse and fine registration, not all possible pairs of combined 2D and depth images are used, but the pairs to be registered may be determined based on a classification of the combined 2D and depth images with respect to a direction relative to the head from which they are captured, for example based on a so-called putative matching graph which indicates for which pairs a registration may be performed. This may be done using approximate pose data or other approximate information to determine combined images for which the poses from which they are captured are similar enough such that a registration is reasonably possible. For example, if one combined image is taken from the left side of the head and another combined image is captured from the right side of the head, there are hardly any common landmark points to be obtained from both images (for example left eye only visible from the left side, right side only visible from the right side, the same for left mouth corner and right mouth corner). Therefore, classification may classify the combined images into categories like left, right, up and down starting from a frontal image. Inside each category, the pairwise coarse and fine registration as described above is performed.

The categorization in some embodiments may be based on metric data from the image recording device itself, for example from ARKit tools in case of iOS based devices or ARCore tools in case of Android based devices used for capturing the combined images. In other cases, the putative matching graph may be derived from the above landmarks via 2D/3D correspondences and a perspective n-point solver, as for example described in Urban, Steffen, Leitloff, Jens and Hinz, Stefan, "Mlpnp - a real-time maximum likelihood solution to the perspective n-point problem," arXiv preprint arXiv:1607.08112 (2016).

In this way, the method may avoid attempting to register combined images for which such a registration is difficult or impossible due to lack of common landmark points which improve robustness.

Based on the registration, i.e., the transformations above, then poses may be estimated for each combined image in a global reference system. The poses may be poses of the head represented by the combined images or camera poses. As explained above, the poses of the camera when capturing the combined images are directly linked to registration of the images and therefore to the poses of the head in the combined images, such that if the camera pose is known, the head pose can be determined and vice versa. This pose graph preferably is then optimized. A possible method for pose graph optimization including generating poses $M_j$ for each of the combined images, based on the registration, is described in Choi, Sungjoon, Zhou, Qian-Yi and Vladlen, Koltun "Robust reconstruction of indoor scenes, proceedings of the IEEE conference on computer vision and pattern recognition, 2015.

Based on these poses $M_j$ a pose graph p = {M, e} is provided, consisting of nodes M, i.e., the poses $M_j$ and edges E, which are the transformations $T_i^j$ and possible covariances $\Sigma_i^J$ if these are determined.

Based on this pose graph, for further optimization then a so-called edge pruning may be performed. This may serve to remove wrong estimates of $T_i^J$ to determine if the poses were estimated from valid edges, i.e., valid transformations obtained in the registration. For this, one of the poses is taken as a reference. Then, edges of the pose graph are concatenated from an unoptimized pose graph along a shortest path to another node. This obtains a further pose estimate for the node to be tested. This further estimate is then compared to the pose from the optimized pose graph. In case of a high deviation in this comparison, i.e., deviation above a threshold, the corresponding edges may be identified as erroneous. These edges may then be removed. Following this, the pose graph optimization mentioned above is repeated without the removed edges, until no more erroneous edges remain in the graph.

Based on the registration and the thus-determined camera poses, then the avatar may be created based on the images essentially in a similar manner as for stationary camera arrangements. This may include fusing the point clouds derived from the individual combined images based on the registration, generating a mesh based on the fused point cloud for example using a Poisson reconstruction as disclosed in Kazhdan, Michael, Bolitho, Matthew and Hoppe, Hugues "Poisson surface reconstruction", Proceedings of the fourth Eurographics symposium on Geometry processing, Vol. 7, 2006 and texturing the mesh based on the images for example as described in Waechter, Michael, Moehrle, Nils and Goesele, Michael, "Let there be color! Large-scale texturing of 3D reconstructions", European conference on computer vision, Springer, Cham, 2014.

Another approach for generating a 3D model of a head using a smartphone is disclosed in WO 2019 / 164502 A1.

The target is provided in the near field. In this way, the near visual point may be determined. The near field is an area where the person uses near vision, which includes typical reading distances or distances for similar tasks, for example a distance up to or at about 40cm from the eyes, as given under 5.28.1 in DIN DIN EN ISO 13666:2012.

The target is the mobile device, e.g., a certain feature of the mobile device like a camera or a target displayed on the mobile device. The relative position and orientation, i.e., pose, of the mobile device (target) with respect to the head when moving the target through the field of view may then be performed using sensors of the mobile device, for example 2D camera and depth sensor as mentioned above. For example, facial landmarks may be detected for each position as described above, and the poses may be detected based on the facial landmarks, corresponding to the coarse registration process described above. Optionally, to refine the poses, a fine registration as described above, and further optionally the pose graph registration as described above may be used. In this way, the poses of the mobile device may be accurately determined.

The plurality of vision points may be provided to a database and displayed as a map, for example on a user interface on the screen of a mobile device or a computer screen as a platform which also provides other information regarding the person's vision like refraction, may enable a virtual try-on of spectacle frames etc. Furthermore, such a platform may provide information on recommended optical design of ophthalmic lenses based on the individual points and provides explanation to an eye care professional or the glasses wearer on optical design. The plurality of visual points recorded at different points in time or using different ophthalmic lens design and or spectacles are stored in the database. The platform may comprise a functionality to directly compare the displayed maps from different points in time or using different ophthalmic lens design and or spectacles from the database.

This information can be used to tailor spectacle lens specifically to the person. Examples for such a tailoring are described in Sheedy, J. E. (2004), "Progressive addition lenses-matching the specific lens to patient needs", Optometry-Journal of the American Optometric Association, 75(2), 83-102, or Sheedy, J., Hardy, R. F., & Hayes, J. R. (2006), "Progressive addition lenses-measurements and ratings", Optometry-Journal of the American Optometric Association, 77(1), 23-39.

Therefore, according to a further aspect, a method for manufacturing spectacle lens is provided, comprising:

> determining a visual point as discussed above, and manufacturing the spectacle lens, for example PAL lens, according to the determined visual point.

In this way, a spectacle lens may be better tailored to the person.

The above concepts will be further explained using specific embodiments, wherein:

> FIG. 1 is a block diagram of a mobile device usable for implementation of embodiments,
> FIG. 2 is a flowchart of a method according to an embodiment,
> FIGs. 3 to 7 are diagrams for explaining the method of FIG. 2, and
> FIGs. 8 and 9 are heat maps showing example visual point distributions.

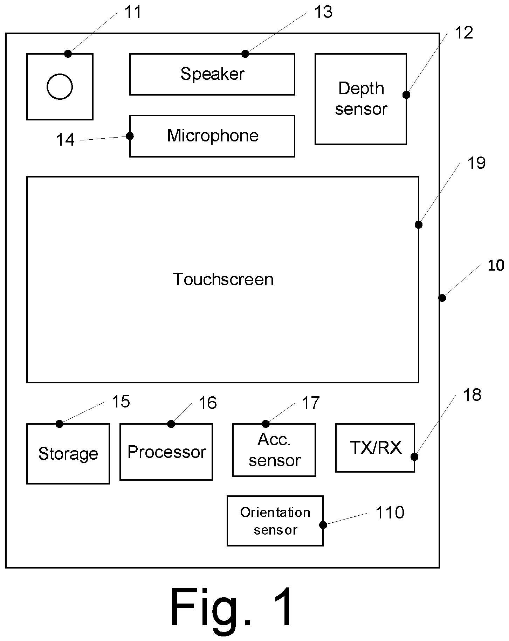

FIG. 1 illustrates a mobile device 10 according to an embodiment. Mobile device 10 is a smartphone or a tablet PC. FIG. 1 shows some components of mobile device 10 that may be used to implement methods as described herein. Mobile device 10 may include further components conventionally used in mobile devices like smartphones, which are not needed for the explanations of embodiments and therefore not shown in FIG. 1.

As sensors, mobile device 10 includes a camera 11 and a depth sensor 12. In many cases, mobile devices include more than one camera, for example a so-called front side camera and a so-called backside camera. Both types of cameras may be used in embodiments. Depth sensor 12 may for example be a time-of-flight based depth sensor, as often provided in mobile devices.

For inputting and outputting information, mobile device 10 includes a speaker 13 for outputting audio signals, a microphone 14 for receiving audio signals and a touchscreen 19. By outputting signals on speaker 13 or displaying information on touchscreen 19, mobile device 10 may output instructions to a person to perform the methods described herein. Via microphone 14 and touchscreen 19, mobile device 10 may receive feedback from a person.

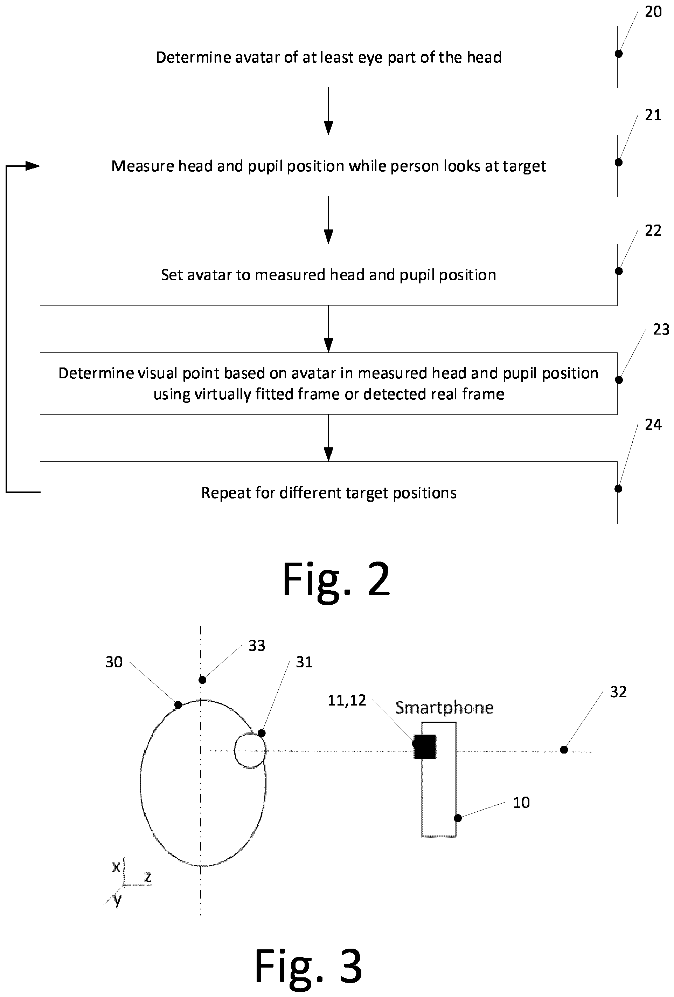

Furthermore, mobile device 10 includes a processor which controls the various components of device 10 and executes programs stored in a storage 15. Storage 15 in mobile devices like smartphones or tablet PCs typically is a flash memory or the like. Storage 15 stores computer programs executed by the processor 16 such that the method described further below with respect to FIG. 2 is executed.

Mobile device 10 further comprises an acceleration sensor 17 and an orientation sensor 110. With acceleration sensor 17, an acceleration of mobile device 10 and, by integrating the acceleration twice, a position of mobile device 10 compared to a start position may be determined. Via orientation sensor 110, an orientation (for example inclination angle) of mobile device 10 may be determined. The combination of position and orientation is also referred to as pose, see DIN EN ISO 8373: 2012-03.

FIG. 2 illustrates a method according to an embodiment, which may be implemented by providing a corresponding computer program in storage 15 of mobile device 10 of FIG. 1 to be executed by processor 16.

In step 20, the method of FIG. 2 comprises determining an avatar of at least an eye portion of the head of a person to be examined. This is further illustrated in FIG. 3. Here, a head 30 of the person with an eye 31 is schematically shown. The person (or another person) holds mobile device 10, for example in this case a smartphone, in front of head 30 while the person looks straight to some target far away, as illustrated by a line 32. The person then rotates the head about the vertical axis 33 while mobile device measures the head using depth sensor 12, and camera 11 for a plurality of rotational positions. From these measurements, then an avatar of at least an eye portion of head 30, i.e., a portion including the eye 31 as shown and the other eye (not shown) and preferably the complete head is calculated by processor 16. Alternatively, mobile device 10 may be moved around head 30, and may measure head 30 at different positions of mobile device 10. Mobile device 10 may determine the positions based on acceleration sensor 17.

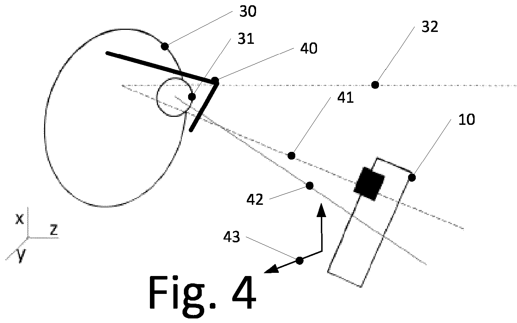

Returning to FIG. 2, at 21 the method comprises measuring the head and pupil position while the person looks at a target. This is illustrated in FIG. 4. In FIG. 4, mobile device 10 serves as the target, for example by displaying some target the person should look at on touchscreen 19. For example, for giving instructions to the person at the same time, mobile device 10 may display a text like "now look here" on touchscreen 19. Mobile device 10 is for example held in a reading position or in another typical near field position. To look at the target, the person inclines head 30 by an angle between line 32 and a line 41. Moreover, the person may rotate eye 13 resulting in a line of sight. Using depth sensor 12 and camera 11, smartphone 10 detects the pupil position and the head position. Additionally, in some embodiments the person may wear a spectacle frame 40, where the position of the spectacle frame is also detected by mobile device 10 as discussed above. In other embodiments, as already explained above, the avatar of the person determined at step 20 may be fitted with a virtual spectacle frame. While in FIG. 4 the target is the mobile device, the person may also look at other targets or at a 3D landscape or scene, and mobile device 10 serves merely for measurements.

At 22, the method comprises setting the avatar to the head and pupil position measured at 21. In other words, the head is sent set to a pose (position and inclination) as measured at 21, and the eyes of the avatar are rotated such that they have the pupil position measured at 21.

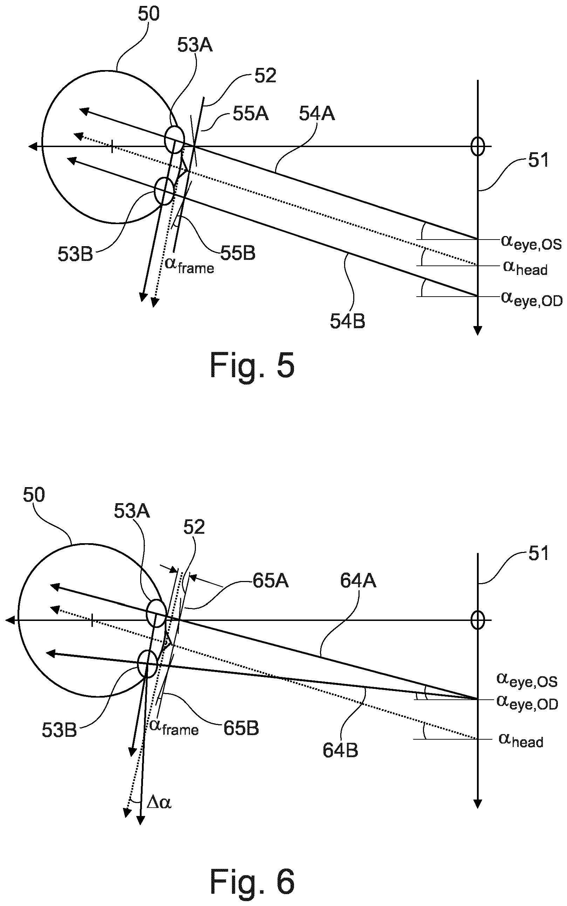

Then, at 23, the method of FIG. 2 includes determining the visual point based on the avatar in the measured head and pupil position. Therefore, unlike some conventional methods, for the final determination no direct measurements are used, but the avatar set to the corresponding position is used as a basis. As mentioned above, for this a real spectacle frame worn by the person or a virtually fitted spectacle frame may be used. This determination will be explained referring to FIGs. 5 to 7.

As indicated by box 24, steps 21 to 23 are repeated for different target positions, for example by moving the mobile device in the field of view of the person as indicated by arrows 43 in Fig. 4. By the repetition, a map of vision points similar to Figs. 8 and 9 may be obtained using a smartphone or similar mobile device. This map may be displayed or transmitted to a lens manufacturer for manufacturing lenses, and then adapted to the vision point map of the respective person.

For the determination of the visual point at 23, mobile device 10, an avatar of head 30, an avatar of eye 31 and spectacle frame 40 (as virtual frame or as detected real frame) may each be represented by a six-dimensional vector including at least three-dimensional position (x, y, z coordinates) and three angles ($\alpha$, $\beta$, $\gamma$). That is, mobile device may be represented by position $\{x_{mobile}, y_{mobile}, z_{mobile}\}$ and an orientation $\{\alpha_{mobile}, \beta_{mobile}, \gamma_{mobile}\}$, the head and/or facial features may be represented by position $\{x_{Head}, y_{Head}, z_{Head}\}$ and an orientation $\{\alpha_{Head}, \beta_{Head}, \gamma_{Head}\}$, the at least one eye may be represented by position $\{x_{eye}, y_{eye}, z_{eye}\}$ and an orientation $\{\alpha_{eye}, \beta_{eye}, \gamma_{eye}\}$. Herein, the position coordinates can be represented in cartesian or polar or similar space. The orientations can be represented in Euler angles or rotation matrix or quaternions or similar. The orientation is measured by orientation sensor 110, and the position of mobile device 10 may be set to 0, such that the other positions explained below are determined relative to mobile device 10.

By measuring for example a few landmark points of head 30, the position of the head of the person $\{x_{head}, y_{head}, z_{head}\}$ and the corresponding orientation $\{\alpha_{head}, \beta_{head}, \gamma_{head}\}$ may be determined. The position and orientation of the eye within the avatar corresponding to eye 31 may be determined as $\{x_{eye}, y_{eye}, z_{eye}\}$ and the orientation may be determined as $\{\alpha_{eye}, \beta_{eye}, \gamma_{eye}\}$. The avatar is then set to the corresponding position and orientation.

Using a mobile device 10, also the center of rotation of the eye may be determined as described for example in European Patent Application EP 20202529.2, and based on the pupil position and the center of rotation the position and orientation of the eye may be determined. Furthermore, also for the spectacle frame a corresponding position $\{x_{frame}, y_{frame}, z_{frame}\}$ and orientation $\{\alpha_{frame}, \beta_{frame}, \gamma_{frame}\}$ may be determined as explained above.

In FIGs. 5 to 7, reference numeral 50 denotes the avatar of the head 30 set to the position and orientation measured in step 21, numerals 53A and 53B designate the left and right eye of the avatar, respectively, and reference 51 designates the position of mobile device 10, i.e., essentially the measurement plane. The position 51 is set to a vertical position for the calculation. This means that the orientations for eye, head and spectacles are transformed based on the measured orientation of mobile device 10 to a coordinate system where mobile device 10 is in an x-z-plane. In FIG. 5, 54A and 54B denote lines of sight of eye 53A and 53B, respectively, and in FIG. 6 lines 64A, 64B denote the respective lines of sight. While registering the relative change of position and orientation of the avatar using at least one camera and/or at least one depth sensor, no additional device is necessary to determine the visual point.

FIGs. 5 to 7 illustrate a rotation of the head about the x-axis of FIGs. 3 and 4, or, in other words, illustrate the y-z-plane. Similar calculations may be performed for other planes.

In FIG. 5, the eyes essentially look straight ahead, such that the angle $\alpha_{eye,OS}$ and $\alpha_{eye,OD}$ corresponding to the left and right eye, respectively, as seen at 51, corresponds to the head rotation angle $\alpha_{head}$. In FIG. 6, the gaze of the two eyes converges at a single point, such that the angles $\alpha_{eye,OS}$ and $\alpha_{eye,OD}$ are different from the angle $\alpha_{head}$ by the respective relative eye rotation angles $\alpha_{eye}$ for the left and right eyes with respect to the head. $\Delta\alpha$ in FIG. 6 designates the difference between the eye rotation and the inclination $\alpha_{frame}$ of the spectacle frame at the respective eye.

In FIG. 5, reference numerals 55A, 55B designate the visual point for the left and right eye, respectively. In FIG. 6, 65A and 65B designate the visual point for the left and right eye. The construction of the visual point based on the angles discussed is again shown in FIG. 7 for a right eye. Essentially, the difference between the spectacle rotation angle and the eye rotation is estimated

by $\Delta\alpha$, and the visual point 70 is then calculated based on the position of the spectacle frame 52 (given for example by the coordinates with index frame mentioned above).

## Claims

1. A computer-implemented method for determining a visual point (55A, 55B; 65A, 65B; 70) of a person by a mobile device (10) including at least one sensor (11, 12), the at least one sensor comprising an image sensor (11), wherein the visual point (55A, 55B; 65A, 65B; 70) is an intersection of a line of sight of the person with a plane approximating the position of a spectacle lens, comprising:

   determining a head position and a pupil position of the person while the person looks at a target using the at least one sensor (11, 12), and determining the visual point (55A, 55B; 65A, 65B; 70) based on an avatar (50) which is a 3D model of at least an eye portion of the head of the person, which is set to the determined head and pupil position, based on the set head and pupil positions of the avatar (50) and on the position of the plane approximating the position of the spectacle lens, wherein either:

   - the method further comprises virtually fitting a spectacle frame to the avatar (50), and determining the visual point (55A, 55B; 65A, 65B; 70) based on the virtually fitted spectacle frame; or
   - the person wears a spectacle frame, and the method further comprises: identifying the spectacle frame worn by the person, wherein determining the visual point (55A, 55B; 65A, 65B; 70) is further based on the identified spectacle frame,

   determining the head position comprises determining parts or facial landmarks of the head of the person, and **characterized in that** the method further comprises moving the target, wherein determining the visual point (55A, 55B; 65A, 65B; 70) is performed for a plurality of target positions, and **in that** the target is the mobile device.

2. The method of claim 1, **characterized in that** determining a head position comprises determining at least one selected from a group consisting of the head position and the facial landmarks relative to the target.

3. The method of claim 1 or 2, **characterized in that** the at least one sensor further comprises a depth sensor (12).

4. The method of claim 3, **characterized in that** determining the head position and the pupil position is performed by using the depth sensor.

5. The method of claim 4, **characterized by** determining the respective poses of the mobile device relative to the head at the plurality of target positions using the depth sensor, and determining the respective visual point for the respective target position based on the respective pose.

6. The method of any one of claims 1 to 5, **characterized by** generating the avatar (50) with the mobile device, wherein generating the avatar comprises:

   providing a plurality of combined 2D and depth images of the head of the person captured from different positions,
   determining landmark points for each of the combined 2D and depth images, and performing a pairwise coarse registration between pairs of combined 2D and depth images based on the landmark points,
   after the pairwise coarse registration, performing a fine registration of the coarse registered combined 2D and depth images based on full point clouds represented by the combined 2D and depth images, and
   generating the avatar (50) based on the registration.

7. The method of claim 6, **characterized in that** providing the plurality of combined 2D and depth images of the head of the person comprises measuring the head of the person in a plurality of positions by the at least one sensor.

8. The method of claim 7, **characterized in that** measuring the head of the person in a plurality of positions comprises measuring the head of the person in a plurality of up-right positions of the head of the person rotated around about an axis of the head.

9. The method of any one of claims 1 to 8 **characterized in that** the target is provided in the near field.

10. A mobile device (10), comprising:

   at least one sensor (11, 12), comprising an image sensor (11) and configured to determine a head position and a pupil position of a person while the person looks at a target, and
   a processor configured to determine the visual point (55A, 55B; 65A, 65B; 70) of the person based on an avatar (50) which is a 3D model of at least an eye portion of the person and set to the determined head and pupil position based on the set head and

pupil positions of the avatar (50) and on a position of a plane approximating the position of a spectacle lens, wherein the visual point (55A, 55B; 65A, 65B; 70) is an intersection of a line of sight of the person with a plane approximating the position of the spectacle lens, wherein either:

- the processor is further configured to virtually fit a spectacle frame to the avatar (50), and determine the visual point (55A, 55B; 65A, 65B; 70) based on the virtually fitted spectacle frame; or
- the person wears a spectacle frame, and the processor is further configured to identify the spectacle frame worn by the person, wherein determining the visual point (55A, 55B; 65A, 65B; 70) is further based on the identified spectacle frame,

determining the head position comprises determining parts or facial landmarks of the head of the person, and **characterized in that** the mobile device (10) is further configured to determine the visual point of the person for a plurality of positions while the target moving through a field of view of the person, and **in that** the target is the mobile device.

11. The mobile device (10) of claim 10, **characterized in that** the mobile device is configured to perform the method of any one of claims 1 to 9.

12. A computer program for a mobile device including at least one sensor (11, 12) and a processor, **characterized in that** the computer program, when executed on the processor, causes execution of the method of any one of claims 1 to 9.

13. A method for producing a spectacle lens, **characterized by**:

determining the visual point (55A, 55B; 65A, 65B; 70) of a person according to the method of any one of claims 1 to 9, and producing the spectacle lens on the basis of the determined visual point (55A, 55B; 65A, 65B; 70).

**Patentansprüche**

1. Computerimplementiertes Verfahren zum Bestimmen eines Sichtpunktes (55A, 55B; 65A, 65B; 70) einer Person durch eine mobile Vorrichtung (10), beinhaltend wenigstens einen Sensor (11, 12), wobei der wenigstens eine Sensor einen Bildsensor (11) umfasst, wobei der Sichtpunkt (55A, 55B;

65A, 65B; 70) ein Schnittpunkt einer Sichtlinie der Person mit einer Ebene ist, die sich der Position eines Brillenglases annähert, umfassend:

Bestimmen einer Kopfposition und einer Pupillenposition der Person, während die Person auf ein Ziel blickt, unter Verwendung des wenigstens einen Sensors (11, 12), und
Bestimmen des Sichtpunktes (55A, 55B; 65A, 65B; 70) basierend auf einem Avatar (50), bei dem es sich um ein 3D-Modell wenigstens eines Augenabschnitts des Kopfes der Person handelt, das auf die bestimmte Kopf- und Pupillenposition eingestellt ist, basierend auf der eingestellten Kopf- und Pupillenposition des Avatars (50) und auf der Position der Ebene, die sich der Position des Brillenglases annähert, wobei entweder:

- das Verfahren ferner das virtuelle Anpassen einer Brillenfassung an das Avatar (50) umfasst, und das Bestimmen des Sichtpunktes (55A, 55B; 65A, 65B; 70) basierend auf der virtuell angepassten Brillenfassung; oder
- die Person eine Brillenfassung trägt und das Verfahren ferner umfasst:

Identifizieren der Brillenfassung, die von der Person getragen wird, wobei das Bestimmen des Sichtpunktes (55A, 55B; 65A, 65B; 70) ferner auf der identifizierten Brillenfassung basiert, wobei das Bestimmen der Kopfposition das Bestimmen von Teilen oder Gesichtslandmarken des Kopfes der Person umfasst und **dadurch gekennzeichnet ist, dass** das Verfahren ferner ein Bewegen des Ziels umfasst, wobei das Bestimmen des Sichtpunktes (55A, 55B; 65A, 65B; 70) für eine Mehrzahl von Zielpositionen durchgeführt wird, und dadurch, dass das Ziel die mobile Vorrichtung ist.

2. Verfahren nach Anspruch 1, **dadurch gekennzeichnet, dass** das Bestimmen einer Kopfposition das Bestimmen von wenigstens einer Position umfasst, die aus einer Gruppe ausgewählt wird, bestehend aus der Kopfposition und den Gesichtslandmarken relativ zum Ziel.

3. Verfahren nach Anspruch 1 oder 2, **dadurch gekennzeichnet, dass** der wenigstens eine Sensor ferner einen Tiefensensor (12) umfasst.

4. Verfahren nach Anspruch 3, **dadurch gekennzeichnet, dass** das Bestimmen der Kopfposition und der Pupillenposition durch Verwenden des Tiefensensors erfolgt.

**5.** Verfahren nach Anspruch 4, **gekennzeichnet durch** das Bestimmen der jeweiligen Posen der mobilen Vorrichtung relativ zum Kopf an einer Mehrzahl von Zielpositionen unter Verwendung des Tiefensensors, und durch das Bestimmen des jeweiligen Sichtpunktes für die jeweilige Zielposition basierend auf der jeweiligen Pose.

**6.** Verfahren nach einem der Ansprüche 1 bis 5, **gekennzeichnet durch** das Generieren des Avatars (50) mit der mobilen Vorrichtung, wobei das Generieren des Avatars umfasst:

Bereitstellen einer Mehrzahl von kombinierten 2D- und -Tiefenbildern des Kopfes der Person, die von unterschiedlichen Positionen aus erfasst werden,
Bestimmen von Landmarkenpunkten für jedes der kombinierten 2D- und Tiefenbilder und
Durchführen einer paarweisen Grobregistrierung zwischen Paaren von kombinieren 2D- und Tiefenbildern basierend auf den Landmarkenpunkten,
Nach der paarweisen Grobregistrierung, Durchführen einer Feinregistrierung der grob registrierten kombinierten 2D- und Tiefenbilder basierend auf vollständigen Punktwolken, dargestellt durch die kombinierten 2D- und Tiefenbilder, und Generieren des Avatars (50) basierend auf der Registrierung.

**7.** Verfahren nach Anspruch 6, **dadurch gekennzeichnet, dass** das Bereitstellen der Mehrzahl von kombinierten 2D- und Tiefenbildern des Kopfes der Person das Messen des Kopfes der Person in einer Mehrzahl von Positionen durch den wenigstens einen Sensor umfasst.

**8.** Verfahren nach Anspruch 7, **dadurch gekennzeichnet, dass** das Messen des Kopfes der Person in einer Mehrzahl von Positionen das Messen des Kopfes der Person in einer Mehrzahl von aufrechten Positionen des Kopfes der Person bei Drehung rund um eine Achse des Kopfes umfasst.

**9.** Verfahren nach einem der Ansprüche 1 bis 8, **dadurch gekennzeichnet, dass** das Ziel im Nahfeld bereitgestellt wird.

**10.** Mobile Vorrichtung (10), umfassend:

wenigstens einen Sensor (11, 12), umfassend einen Bildsensor (11) und dazu ausgelegt, eine Kopfposition und eine Pupillenposition einer Person zu bestimmen, während die Person auf ein Ziel blickt, und
einen Prozessor, der dazu ausgelegt ist, den Sichtpunkt (55A, 55B; 65A, 65B; 70) der Person basierend auf einem Avatar (50) zu bestimmen, bei dem es sich um ein 3D-Modell wenigstens eines Augenabschnitts der Person handelt und das auf die bestimmte Kopf- und Pupillenposition eingestellt ist, basierend auf der eingestellten Kopf- und Pupillenposition des Avatars (50) und auf einer Position einer Ebene, die sich der Position eines Brillenglases annähert, wobei der Sichtpunkt (55A, 55B; 65A, 65B; 70) ein Schnittpunkt einer Sichtlinie der Person mit einer Ebene ist, die sich der Position des Brillenglases annähert, wobei entweder:

- der Prozessor ferner dazu ausgelegt ist, eine Brillenfassung virtuell an das Avatar (50) anzupassen und den Sichtpunkt (55A, 55B; 65A, 65B; 70) basierend auf der virtuell angepassten Brillenfassung zu bestimmen; oder
- die Person eine Brillenfassung trägt und der Prozessor ferner dazu ausgelegt ist, die von der Person getragene Brillenfassung zu identifizieren, wobei das Bestimmen des Sichtpunktes (55A, 55B; 65A, 65B; 70) ferner auf der identifizierten Brillenfassung basiert,

wobei das Bestimmen der Kopfposition das Bestimmen von Teilen oder Gesichtslandmarken des Kopfes der Person umfasst und **dadurch gekennzeichnet ist, dass** die mobile Vorrichtung (10) ferner dazu ausgelegt ist, den Sichtpunkt der Person für eine Mehrzahl von Positionen zu bestimmen, während sich das Ziel durch ein Sichtfeld der Person bewegt, und dadurch, dass das Ziel die mobile Vorrichtung ist.

**11.** Mobile Vorrichtung (10) nach Anspruch 10, **dadurch gekennzeichnet, dass** die mobile Vorrichtung dazu ausgelegt ist, das Verfahren nach einem der Ansprüche 1 bis 9 durchzuführen.

**12.** Computerprogramm für eine mobile Vorrichtung, die wenigstens einen Sensor (11, 12) und einen Prozessor aufweist, **dadurch gekennzeichnet, dass** das Computerprogramm, wenn es auf dem Prozessor ausgeführt wird, die Ausführung des Verfahren nach einem der Ansprüche 1 bis 9 veranlasst.

**13.** Verfahren zum Herstellen eines Brillenglases, **gekennzeichnet durch**:

Bestimmen des Sichtpunktes (55A, 55B; 65A, 65B; 70) einer Person gemäß dem Verfahren nach einem der Ansprüche 1 bis 9 und Herstellen des Brillenglases auf Basis des bestimmen Sichtpunktes (55A, 55B; 65A, 65B; 70).

## Revendications

1. Procédé mis en œuvre par ordinateur pour la détermination d'un point visuel (55A, 55B ; 65A, 65B ; 70) d'une personne par un dispositif mobile (10) comportant au moins un capteur (11, 12), l'au moins un capteur comprenant un capteur d'image (11), le point visuel (55A, 55B ; 65A, 65B ; 70) étant une intersection d'une ligne de visée de la personne avec un plan se rapprochant de la position d'un verre de lunettes, comprenant :

   la détermination d'une position de la tête et d'une position de la pupille de la personne pendant que la personne regarde une cible au moyen de l'au moins un capteur (11, 12), et la détermination du point visuel (55A, 55B ; 65A, 65B ; 70) sur la base d'un avatar (50) qui est un modèle 3D d'au moins une portion d'œil de la tête de la personne, qui est placé à la position de la tête et de la pupille déterminée, sur la base des positions de la tête et de la pupille établies de l'avatar (50) et de la position du plan se rapprochant de la position du verre de lunettes, dans lequel :

      - le procédé comprend en outre l'ajustement virtuel d'une monture de lunettes sur l'avatar (50), et la détermination du point visuel (55A, 55B ; 65A, 65B ; 70) sur la base de la monture de lunettes ajustée virtuellement ; ou
      - la personne porte une monture de lunettes, et le procédé comprend en outre : l'identification de la monture de lunettes portée par la personne, dans lequel la détermination du point visuel (55A, 55B ; 65A, 65B ; 70) est également basée sur la monture de lunettes identifiée,

   la détermination de la position de la tête comprend la détermination de parties ou de repères faciaux de la tête de la personne, et **caractérisé en ce que** le procédé comprend en outre le déplacement de la cible, dans lequel la détermination du point visuel (55A, 55B ; 65A, 65B ; 70) est réalisée pour une pluralité de positions de la cible, et **en ce que** la cible est le dispositif mobile.

2. Procédé selon la revendication 1, **caractérisé en ce que** la détermination d'une position de la tête comprend la détermination d'au moins un élément choisi dans un groupe constitué par la position de la tête et les repères faciaux par rapport à la cible.

3. Procédé selon la revendication 1 ou 2, **caractérisé en ce que** l'au moins un capteur comprend également un capteur de profondeur (12).

4. Procédé selon la revendication 3, **caractérisé en ce que** la détermination de la position de la tête et de la position de la pupille est effectuée au moyen du capteur de profondeur.

5. Procédé selon la revendication 4, **caractérisé par** la détermination des poses respectives du dispositif mobile par rapport à la tête à la pluralité de positions de la cible au moyen du capteur de profondeur, et la détermination du point visuel respectif pour la position de la cible respective sur la base de la pose respective.

6. Procédé selon l'une quelconque des revendications 1 à 5, **caractérisé par** la génération de l'avatar (50) avec le dispositif mobile, dans lequel la génération de l'avatar comprend :

   l'obtention d'une pluralité d'images 2D et de profondeur combinées de la tête de la personne capturées depuis différentes positions,
   la détermination de points de repère pour chacune des images 2D et de profondeur combinées, et la réalisation d'un alignement par paires grossier entre paires d'images 2D et de profondeur combinées sur la base des points de repère,
   après l'alignement par paires grossier, la réalisation d'un alignement fin des images 2D et de profondeur combinées grossièrement alignées sur la base de nuages de points complets représentés par les images 2D et de profondeur combinées, et
   la génération de l'avatar (50) sur la base de l'alignement.

7. Procédé selon la revendication 6, **caractérisé en ce que** l'obtention de la pluralité d'images 2D et de profondeur combinées de la tête de la personne comprend la mesure de la tête de la personne dans une pluralité de positions par l'au moins un capteur.

8. Procédé selon la revendication 7, **caractérisé en ce que** la mesure de la tête de la personne dans une pluralité de positions comprend la mesure de la tête de la personne dans une pluralité de positions droites de la tête de la personne tournées autour d'un axe de la tête.

9. Procédé selon l'une quelconque des revendications 1 à 8 **caractérisé en ce que** la cible est disposée dans le champ proche.

10. Dispositif mobile (10), comprenant :

   au moins un capteur (11, 12), comprenant un capteur d'image (11) et conçu pour déterminer une position de la tête et une position de la pupille d'une personne pendant que la personne regarde une cible, et

un processeur conçu pour déterminer le point visuel (55A, 55B ; 65A, 65B ; 70) de la personne sur la base d'un avatar (50) qui est un modèle 3D d'au moins une portion d'œil de la personne et placé à la position de la tête et de la pupille déterminée sur la base des positions de la tête et de la pupille établies de l'avatar (50) et de la position d'un plan se rapprochant de la position d'un verre de lunettes, le point visuel (55A, 55B ; 65A, 65B ; 70) étant une intersection d'une ligne de visée de la personne avec un plan se rapprochant de la position du verre de lunettes, dans lequel :

- le processeur est également conçu pour ajuster virtuellement une monture de lunettes sur l'avatar (50), et déterminer le point visuel (55A, 55B ; 65A, 65B ; 70) sur la base de la monture de lunettes ajustée virtuellement ; ou
- la personne porte une monture de lunettes, et le processeur est également conçu pour identifier la monture de lunettes portée par la personne, dans lequel la détermination du point visuel (55A, 55B ; 65A, 65B ; 70) est également basée sur la monture de lunettes identifiée,

la détermination de la position de la tête comprend la détermination de parties ou de repères faciaux de la tête de la personne, et **caractérisé en ce que** le dispositif mobile (10) est également conçu pour déterminer le point visuel de la personne pour une pluralité de positions pendant que la cible se déplace à travers un champ de vision de la personne, et **en ce que** la cible est le dispositif mobile.

11. Dispositif mobile (10) selon la revendication 10, **caractérisé en ce que** le dispositif mobile est conçu pour réaliser le procédé de l'une quelconque des revendications 1 à 9.

12. Programme informatique pour un dispositif mobile comportant au moins un capteur (11, 12) et un processeur, **caractérisé en ce que** le programme informatique, lorsqu'il est exécuté sur le processeur, provoque l'exécution du procédé de l'une quelconque des revendications 1 à 9.

13. Procédé de production d'un verre de lunettes, **caractérisé par** :

la détermination du point visuel (55A, 55B ; 65A, 65B ; 70) d'une personne selon le procédé de l'une quelconque des revendications 1 à 9, et la production du verre de lunettes sur la base du point visuel déterminé (55A, 55B ; 65A, 65B ; 70).

Fig. 1

20

Determine avatar of at least eye part of the head

21

Measure head and pupil position while person looks at target

22

Set avatar to measured head and pupil position

23

Determine visual point based on avatar in measured head and pupil position using virtually fitted frame or detected real frame

24

Repeat for different target positions

# Fig. 2

30    33    31

11,12    Smartphone    32

10

X
Z
y

# Fig. 3

Fig. 4

Fig. 5

Fig. 6

Fig. 7

horizontal position [mm]

vertical position [mm]

Fig. 8

Fig. 9

## REFERENCES CITED IN THE DESCRIPTION

**Patent documents cited in the description**

- EP 3145386 B1
- WO 2015124574 A1
- EP 1815289 B9
- EP 1747750 A1
- EP 2342599 B1
- EP 1960826 B1
- US 2020349754 A1
- US 2021142566 A1
- US 9645413 B2
- US 9841615 B2
- EP 3413122 A1
- US 2021181538 A1
- EP 3542211 A1
- US 20030123026 A1
- US 2002105530 A1
- US 20160327811 A1
- WO 2019164502 A1
- EP 20202529

**Non-patent literature cited in the description**

- **WU, Y.** ; **HASSNER, T.** ; **KIM, K.** ; **MEDIONI, G.** ; **NATARAJAN, P.** Facial landmark detection with tweaked convolutional neural networks. *IEEE transactions on pattern analysis and machine intelligence*, 2017, vol. 40 (12), 3067-3074
- **PERAKIS, P.** ; **PASSALIS, G.** ; **THEOHARIS, T.** ; **KAKADIARIS, I. A.** 3D facial landmark detection under large yaw and expression variations. *IEEE transactions on pattern analysis and machine intelligence*, 2012, vol. 35 (7), 1552-1564
- **WU, Y.** ; **JI, Q.** Facial landmark detection: A literature survey. *International Journal of Computer Vision*, 2019, vol. 127 (2), 115-142
- **RAU J-Y** ; **YEH P-C**. A SemiAutomatic Image-Based Close Range 3D Modeling Pipeline Using a Multi-Camera Configuration. *Sensors (Basle, Switzerland)*, 2012, vol. 12 (8), 11271-11293, 11289
- **M. NIEßNER** ; **M. ZOLLHÖFER** ; **S. IZADI** ; **M. STAMMINGER**. Real-time 3D reconstruction at scale using voxel hashing. *ACM Trans. Graph.*, November 2013, vol. 32 (6)
- **WU, Y.** ; **JI, Q.** Facial landmark detection: A literature survey. *International Journal of Computer Vision*, 2018, vol. 127 (2), 115-142
- Method for registration of 3D shapes. **BESL, PAZL J.** ; **MCKAY, NEIL D.** Sensor fusion IV: control paradigms and data structures. International Society for Optics and Photonics, 1992, vol. 1611
- **FISCHLER, MARTIN A.** ; **BOLLES, ROBERT C.** Random sampling consensus: a paradigm for model fitting with applications to image analysis and automated cartography. *Communications of the ACM*, 1981, vol. 24 (6), 381-2395
- **PARK, JAESIK** ; **ZHOU, QUIAN-YI** ; **KOLTUN, VLADLEN**. Colored point cloud registration revisited. *Proceedings of the IEEE International Conference on Computer Vision*, 2017
- Efficient variants of the ICP algorithm. **RUSINKIEWICZ, SZYMON** ; **LEVOY, MARC**. Proceedings of the third international conference on 3D digital imaging and modeling. IEEE, 2001
- **URBAN, STEFFEN** ; **LEITLOFF, JENS** ; **HINZ, STEFAN**. Mlpnp - a real-time maximum likelihood solution to the perspective n-point problem. *arXiv preprint arXiv:1607.08112*, 2016
- **CHOI, SUNGJOON** ; **ZHOU, QIAN-YI** ; **VLADLEN, KOLTUN**. *Robust reconstruction of indoor scenes, proceedings of the IEEE conference on computer vision and pattern recognition*, 2015
- **KAZHDAN, MICHAEL** ; **BOLITHO, MATTHEW** ; **HOPPE, HUGUES**. Poisson surface reconstruction. *Proceedings of the fourth Eurographics symposium on Geometry processing*, 2006, vol. 7
- Let there be color! Large-scale texturing of 3D reconstructions. **WAECHTER, MICHAEL** ; **MOEHRLE, NILS** ; **GOESELE, MICHAEL**. European conference on computer vision. Springer, 2014
- **SHEEDY, J. E.** Progressive addition lenses-matching the specific lens to patient needs. *Optometry-Journal of the American Optometric Association*, 2004, vol. 75 (2), 83-102
- **SHEEDY, J.** ; **HARDY, R. F.** ; **HAYES, J. R.** Progressive addition lenses-measurements and ratings. *Optometry-Journal of the American Optometric Association*, 2006, vol. 77 (1), 23-39
- *DIN EN ISO 8373*, March 2012